# EUROPÄISCHE PATENTSCHRIFT

(11) **EP 0 366 156 B1**
(45) Veröffentlichungstag und Bekanntmachung des Hinweises auf die Patenterteilung: **11.01.1995**
(21) Anmeldenummer: 89120921.5
(22) Anmeldetag: 25.01.1985
(51) Int. Cl.: A61K 31/355

(54) **Mittel zur Behandlung von Erkrankungen der Venen und des Analbereichs**
Composition for the treatment of diseases of the veins and the anal region
Composition pour le traitement des maladies de veines et de la région anale

(30) Priorität: 28.01.1984 DE 3402930; 15.02.1984 DE 3405240; 27.02.1984 DE 3407024; 27.02.1984 DE 3407026; 07.03.1984 DE 3408260; 24.04.1984 DE 3415250; 02.05.1984 DE 3416162; 17.07.1984 DE 3427193; 07.09.1984 DE 3432881
(43) Veröffentlichungstag der Anmeldung: 02.05.1990
(62) Teilanmeldung aus: 85100749.2
(73) Patentinhaber: Ismail, Roshdy, Dr., D-50939 Köln (DE)
(72) Erfinder: Ismail, Roshdy, Dr., D-50939 Köln (DE)
(74) Vertreter: Werner, Hans-Karsten, Dr.Dipl.-Chem.

(56) Entgegenhaltungen:
- DE-A- 2 425 222
- DICTIONNAIRE VIDAL, 1982, edition 58, OVP, page 950, Paris, FR
- ROTE LISTE, 1983, Seite 151, Editio Cantor, Aulendorf/Württ, DE
- ROTE LISTE 1981, 52215 Salus (R) Herz-Schutz-Kapseln
- ROTE LISTE 1983, 83124 Eusovit (R) 300

## Beschreibung

Gegenstand der vorliegenden Erfindung ist die Verwendung von Vitamin E gemäß Anspruch 1.

Vitamin E ist bekannt als Antioxidans und Schutzvitamin für Phospholipide der Zellmembran. Es hält die Permeabilität und Stabilität der Zellmembran aufrecht; Lucy, Ann. N.Y. Academy of Science 203 1972, S. 4. Es ist weiterhin bekannt, daß Vitamin E eine membranabdichtende Wirkung besitzt; F. Mittelbach und G. Bodechtel, Münchner Medizinische Wochenschrift 110 (1968) 36, S. 1988-1993. Bei Erythrocyten, den einfachsten Zellen des menschlichen Körpers wurde festgestellt, daß Vitamin E eine Schutzwirkung für die Zellmembran darstellt. In Tierversuchen und beim Menschen wurde bewiesen, daß Anämie das erste Anzeichen von Vitamin-E-Mangel ist. Bei Gabe von hohen Vitamin-E-Dosen normalisiert sich die Hämolyse der Erythrocyten; vgl. William J. Darbey Vitamin Horm, 26 (50) S. 685-704 (1968) und Phelps DL Pediatrics 63 (6) S. 933-935 (1979). Aus diesen Literaturstellen geht hervor, daß bei Gabe von 200 bis 800 mg Vitamin E oral für einen Zeitraum von 1 bis 4 Tagen, die Hämolyse der Erythrocyten significant verbessert wird im Vergleich zu Patienten mit Vitamin-E-Mangel.

Vitamin E ist weiterhin verwendet worden zur Behandlung der Sichelzellenanämie in einem Zeitraum von 6 bis 35 Wochen; vgl. Natt CL. Am. J. Clin. 33, S. 968-971 (1980); Natt CL. Am. J. Clin. nutr. 32, S. 1359-1362 (1979); Gawlik G.M. Fed. Proc. 35 (3), S. 252 (1976) und Gorash L. Bieri J.G. et al univ. Conn. Farmington, GT.

Weiterhin ist bekannt, daß 750 mg Vitamin E täglich in einem Zeitraum von 3 bis 6 Monaten erfolgreich bei Thalassamie-Patienten eingesetzt wurde, wobei eine Normalisierung der Hämolyse der Erythrocyten beobachtet wurde; vgl. Kahane I. ISR. J. med. 12 (1), S. 11-15 (1976).

Erfolgreich eingesetzt wurde Vitamin E weiterhin bei Patienten mit akuter Hepatitis und alkoholischer Hepatitis, die einen Mangel an Vitamin E im Serum haben; vgl. Yoshiakawa T. Takemura S. Kato H. et al. Japan J. Gastrovent, 74/7, S. 732-739 (1977). Schließlich wurde Vitamin E bei Patienten mit Eisenmangelanämie eingesetzt und bewirkte während eines Zeitraumes von 4 bis 8 Wochen eine Verbesserung bzw. Normalisierung des Lipidmetabolismus im Knochenmark; vgl. Takoshi Itaga, Central Clinical Laboratory Nagasaki University of Medicine, Japan.

Es ist weiterhin bekannt, daß Benzaronsalbe und Tabletten zur Behandlung von Beinvenenerkrankungen und Krampfadern sowie damit verbundenen Störungen verwendet werden. Ein Nachteil dieses Wirkstoffes ist, daß bei Lichteinwirkungen Hautreaktionen, Photosensibilität sowie Allergien auftreten.

In B. Helwig, Moderne Arzneimittel, 5. Auflage S. 1446 bis 1447 (1980) ist ausgeführt, daß der Wirkungsmechanismus des Vitamin E nur zum Teil geklärt ist. Insbesondere greift danach Vitamin E in den Umsatz und die Biosynthese von Kohlenhydraten, Eiweißkörpern, Kreatin- und Nukleinsäuren ein. Die Beschleunigung der Gewebereinigung bzw. -entgiftung durch hohe Vitamin-E-Dosen wurde ebenfalls beobachtet. Bestimmte Vitamin-E-Konzentrationen und Kombinationen von Vitamin E mit anderen Wirkstoffen wurden jedoch nicht untersucht.

Aus WO 83/01898 ist eine pharmazeutische Wirkstoffkombination bekannt, die Vitamin A, Vitamin E, Mandelöl, Sesamöl und Olivenöl enthält. Der Nachteil einer derartigen Zusammensetzung liegt darin, daß Vitamin E schlecht von der Haut aufgenommen wird. Außerdem hat Vitamin E mit Sicherheit keine Wirkung, da es lediglich in niedrigen Konzentrationen zugesetzt wird.

Die DE-24 25 222 A1 betrifft pharmazeutische Zubereitungen, welche Vitamine E und Vincamin bzw. Vitamin E-, Vincamin- und Vitamin-P-Faktoren, beispielsweise Rutin-Derivate, enthalten. Diese Arzneimittel werden in Gelatinekapseln, die ungefähr 200 mg Trioxyäthylrutin, 8 bis 10 mg Vincamin und 100 mg α-Tocopherolacetat enthalten, verabreicht. Diese Wirkstoffkombination wurde zur Behandlung von Venenentzündungen eingesetzt. Als weitere Indikationen werden genannt, thrombo-embolische Erkrankungen, Arteriosklerose, Cerebralinsuffizienz, Cerebralsklerose, Myopie, Netzhauterkrankungen sowie cerebral-okulare Alterungserscheinungen.

Das Dictionnaire Vidal 1982 beschreibt unter der Spezifikation eines Produkts mit Handelsname Pariéval die Kapseln mit einem Gehalt von 100 mg Tocopherolacetat, 200 mg Troxerutin und 8 mg Vincamin. Die Kapseln werden zur Behandlung von cochleo-vestibulairen Störungen wie Schwindel, bei Retinopathien vasculairen Ursprungs, Alterserscheinungen und bei klinischen Manifestationen venöser Insuffizienz und Brüchigkeit der Kapillaren eingesetzt. Als Dosierung werden 3 bis 4 Kapseln täglich, in besonderen Fällen bis zu 6 Kapseln täglich angegeben.

Die Rote Liste 1983, Delta Pimafucort® offenbart ein Kombinationspräparat enthaltend Vitamin E, Vitamin A, Dexamethason-21-isonicotinat und weitere Wirkstoffe.

Die Rote Liste 1981, Salus® Herz-Schutz-Kapseln offenbart ein Kombinationspräparat in Kapselform, welches Vitamin E, Rutin und weitere Wirkstoffe enthält.

Die Rote Liste 1983, Eusovit® 300 offenbart Kapseln enthaltend DL-α-Tocopherolacetat in Mengen von 300 mg. Das Präparat wird bei Erkrankungen des Stütz- und Muskelgewebes, Lumbago, Bandscheibenschäden, Myalgien, progressiver Muskeldystrophie, Koronar- und peripheren Gefäßerkrankungen, Artherosklerosen, Fertilitätsstörungen sowie zur Verminderung der Digitalistoxizität empfohlen.

Es wurde erfindungsgemäß überraschenderweise gefunden, daß die Verwendung von Vitamin E im Bereich von 200 bis 600 mg pro Darreichungseinheit in Kombination mit Extractum Hippocastani oder β-Hydroxyethylrutosid als alleiniger Wirkstoffkombination sowie gegebenenfalls üblichen Trägern und Hilfsstoffen zur Herstellung eines Mittels zur Behandlung von Erkrankungen der Venen und zur Verbesserung der Durchblutung der Extremitäten, im Wege der oralen Verabreichung geeignet sind. Dieser neue Indikationsbereich war aufgrund des bisherigen Wissenstandes nicht vorherzusehen und eröffnet ein neues Anwendungsfeld für Vitamin E.

Unter Venenerkrankungen versteht man variköses postthrombotisches Syndrom. Typische Indikationen sind somit Krampfadern mit den Anzeichen Schmerzen, nächtliche Beinkrämpfe und Schwellung. Auch chronische venöse Insuffizienz kann erfindungsgemäß schneller gelindert werden und bringt auf lange Sicht wesentliche Dauerverbesserung.

Die erfindungsgemäße Verwendung von Vitamin E verkürzt die Behandlungszeit. Die Symptome gehen schneller zurück. Die Verwendung der Vitamin-E-haltigen Kombinationspräparaten muß jedoch auf längere Zeit, ca. 6 Monate oder länger, erfolgen.

Diese Ergebnisse waren nicht vorhersehbar und ermöglichen eine Therapie, bei der ein Teil des chemischen Wirkstoffes durch einen Naturstoff ersetzt wird, der sich obendrein praktisch in jeder Körperzelle befindet.

Die erfindungsgemäße Verwendung von Vitamin E ist zur Behandlung von Krankheiten im Analbereich geeignet. Hier tritt auch nach deren Einnahme eine schnelle Linderung und Beseitigung der Symptome von Hämorrhoidalerkrankungen ein.

Entscheidend für die Wirksamkeit von Vitamin E bei der Kombination mit gefäßerweiternden und/oder durchblutungsfördernden Mitteln ist vor allem eine ausreichende Dosierung, die mindestens 200 mg betragen sollte. Niedrigere Dosierungen an Vitamin E sind nutzlos, da große Teile durch die Magensäure zerstört werden und dadurch ihre Wirksamkeit verlieren; vgl. Arthur Vogelsang in Angiology 21, S. 275-279 (1970).

Sofern in der Vergangenheit hin und wieder geringe Mengen Vitamin E, nämlich bis zu 40 mg in Kombinationspräparaten zum Einsatz gekommen sind, waren sie mit Sicherheit wegen der zu niedrigen Dosierung völlig wirkungslos. Zur Behandlung von Venenerkrankungen sollte die Dosierung vorzugsweise im Bereich 300 bis 500 mg pro Darreichungseinheit sein. Typische Kombinationspräparate enthalten 300 bis 400 mg Vitamin E.

Als Vitamin E bei der oralen Darreichungsform kann sowohl der Ester aus natürlicher oder synthetischer Herkunft als auch das freie Tocopherol verwendet werden.

Außer der Wirkstoffkombination enthalten die zu verwendenden Mittel übliche Träger- und Hilfsstoffe. Da Vitamin E bei üblichen Temperaturen flüssig ist, bietet sich hierfür als Applikationsform insbesondere die Weichgelatinekapsel an.

Die Wirkstoffe werden in Vitamin E sowie gewünschtenfalls in einem dünnflüssigen Neutralöl und einem Lösungsvermittler in an sich bekannter Weise in Weichgelatinekapseln eingebracht, auch hier können geeignete Emulgatoren, wie z.B. Tween®, eingesetzt werden. Hierbei können insbesondere die Standardrezepturen der Firma Scherer, Eberbach, zur Anwendung kommen. Die Verwendung dieser Kombinationen in Form von Tropfen, z.B. als alkoholische Lösung, kann ebenfalls geeignet sein.

Überraschenderweise wurde ferner gefunden, daß hochdosiertes Vitamin E mit durchblutungsfördernden Mitteln oder Derivate als Venenmittel besondere Vorteile bringt, wenn Vitamin A zugesetzt wird. Hierbei wird insbesondere die Behandlungsdauer verkürzt. Diese Erfindung beschreibt daher weiterhin die Verwendung von Vitamin A und E und Extractum hippocastani bzw. β-Hydroxyethylrutosid.

Vitamin A und E neigen insbesondere in Gegenwart von anderen Wirkstoffen im wäßrigen Medium sehr stark zu Klumpenbildung. Daher besteht die Gefahr, daß die fettlöslichen wertvollen Stoffe nicht absorbiert werden.

Überraschend wurde festgestellt, daß geringe Mengen Emulgator, ca. 1% ausreichen, um die Klumpenbildung zu verhindern. Die Wirkstoffe werden leichter im wäßrigen Medium dispergiert bzw. suspendiert. Dies hat den Vorteil, daß die Absorption durch den Darm erleichtert wird. Eine größere Menge Emulgator ist nicht notwendig, da meistens 1 bis 5% ausreichend sind, um die Klumpenbildung zu verhindern. Man kann auch Emulgatoren bis zu 10% oder mehr verwenden. Der Nachteil ist aber, daß bei Zugabe großer Mengen und der Einnahme des Medikaments über lange Zeiträume sich eventuell Nebenwirkungen zeigen. Es können die üblichen Emulgatoren, die in den medizinischen Präparaten verwendet werden, wie Tween®20, Cremophore®, aliphatische Alkohole etc. verwendet werden. Im Rahmen der erfindungsgemäßen Verwendung werden jedoch Tween®80 und Cetiol bevorzugt.

Man kann als Emulgator auch Lecithin in einer Konzentration zwischen 1 und 13% verwenden. Damit wird die Resorption von der Kombination A + E, insbesondere von A begünstigt. Geringe Mengen des Emulgators aus Lecithin sind ausreichend, um die Klumpenbildung der fettlöslichen Vitamine zu verhindern und um eine optimale Resorption zu begünstigen. Auch die Verwendung von großen Mengen Lecithin, bis zu 50%, zeigt positive Wirkung. Jedoch ist zu empfehlen, ca. 1% herkömmliche Emulgatoren wie Tween®80 zuzusetzen. Dadurch wird die Mischbarkeit von Lecithin mit den beiden Vitaminen begünstigt und eine Klumpenbildung verhindert.

Besonders vorteilhaft ist hinsichtlich der Resoption die Verwendung von herkömmlichen Emulgatoren wie z.B. ca. 1% Tween®80 zusammen mit 1 bis 13% Lecithin. Statt Tween®80 können auch als Emulgatoren Tween®20, Cetiol, Ölsäureoleylester, Cremophore® verwendet werden. Es wird als Lecithinpräprarat das Sojalecithin bevorzugt verwendet.

Vitamin A kann als Vitamin-A-Palmitat, als auch Vitamin-A-Acetat, als auch weiterer Ester des Vitamin A, als auch β-Carotin verwendet werden.

Vitamin E kann in allen seinen α-Formen verwendet werden, sowohl als freies als auch als Ester. Dieser Ester kann als Acetat, Succinat oder als anderer Ester verwendet werden. Man kann auch andere Darreichungsformen zubereiten, wie Tabletten oder Dragées, wenn man Vitamin E in fester Form verwendet. Auch als alkoholische Lösung ist es zu verwenden. Die Menge des Vitamin E soll möglichst hoch dosiert sein, zwischen 200 bis 600 mg, vorzugsweise zwischen 300 bis 500 mg pro Darreichungsform. Vitamin A soll so ausgewählt sein, daß die maximale Tagesdosis 50.000 I.E. nicht überschreitet, d.h. wenn zwei Darreichungsformen pro Tag angewandt werden, soll die Dosierung bei maximal 25.000 I.E. pro Darreichungsform liegen. Es können auch weitere Zusätze wie Vitamine, z.B. Vitamine der B-Reihe oder Analgetika etc. zugesetzt werden. Als durchblutungsfördernde Mittel werden β-Hydroxyäthylrutosid und Extract Hippocastani eingesetzt.

Diese Stoffe können mit Vitamin A und E kombiniert, außerdem gegen Arteriosklerose eingesetzt werden.

Die durchblutungsfördernden Mittel können ebenfalls in Retard-Form verwendet werden.

In den Kapseln muß Vitamin E in genügend hoher Dosierung vorhanden sein, und zwar pro Kapsel besonders bevorzugt zwischen 400 bis 500 mg.

Vitamin E ist überraschenderweise auch hervorragend zur Behandlung von Erkrankungen des Analbereiches geeignet. Insbesondere eignet es sich zur Behandlung von Hämorrhoiden, Varizen, altersbedingter Venenschwäche, Kapillaropathie, Thrombophlebitis im Analbereich, Pruritus ani, Analfissuren, Analrhagaden und feuchten Analexzemen.

Dieser neue Indikationsbereich war aufgrund des bisherigen Wissensstandes nicht vorherzusehen und eröffnet ein neues und breites Anwendungsfeld für Vitamin E.

Prinzipiell ist es möglich, Vitamin E bei diesen Indikationen oral zu applizieren, so daß alle bisher bekannten oralen Applikationsformen verwendet werden können. Besonders geeignet sind Weichgelatine-Kapseln, die entweder α-Tocopherol aus Soja-, Mais,- oder Weizenkeimlingen, oder aber D,L-α-Tocopherolacetat enthalten.

Die erfindungsgemäße Verwendung des Vitamin E ist aus zwei Gründen besonders bevorzugt: Mit ihrer Hilfe ist es möglich, das Vitamin E verzögerungsfrei und in relativ hohen Konzentrationen in den gewünschten Bereich zur Verfügung zu stellen. Weiterhin werden die Verluste an Vitamin E bei oraler Applikation vermieden, da bekannt ist, daß Vitamin E in erheblichem Maße durch Magensäure zerstört wird.

Zur Behandlung der Erkrankung des Analbereiches wird Vitamin E anfangs mehrmals täglich, später nur noch einmal täglich angewendet.

Bei oraler Anwendung sollte die Dosierung mindestens bei 200 bis 400 mg Vitamin E liegen, da bekannt ist, daß ein Teil des Vitamin E durch Magensäure wieder zerstört wird. Als Vitamin E kommt sowohl natürliches D-α-Tocopherol und seine Konzentrate oder synthetisches D,L-α-Tocopherol in Frage.

Die Kombinationen mit Extract Hippocastani oder β-Hydroxyäthylrutosid werden verwendet.

### Beispiel 1

### Kapseln enthalten:

200 mg β-Hydroxyäthyl-rutosid;
400 mg D-α-Tocopherolacetat;
180 mg Sojaöl;

### Beispiel 2

### Kapseln enthalten:

150 mg Extract Hippocastani (enthalten 25 mg Aescin);
400 mg D-α-Tocopherol;
150 mg Sojaöl;

### Beispiel 3

### Kapseln enthalten:

150 mg Extract Hippocastani (enthalten 25 mg Aescin);
350 mg Vitamin E;
150 mg Sojaöl;

### Beispiel 4

### Tropfen

100 ml 90% Äthylalkohol enthält:
40 g D,L-α-Tocopherolacetat;
4,5 g Extract Hippocastani (enthalten 750 mg Aescin);

### Beispiel 5

| Kapseln enthaltend | |
|---|---|
| DL-α-Tocopherol | 400 mg |
| β-Hydroxyäthylrutosid | 300 mg |
| Vitamin A Palmitat | 30.000 I.E. |
| Sojaöl | 150 mg |

### Beispiel 6

| Kapseln enthaltend | |
|---|---|
| DL-α-Tocopherolacetat | 400 mg |
| β-Hydroxyäthylrutosid | 300 mg |
| Vitamin A Palmitat | 25.000 I.E. |
| Sojaöl | 120 mg |

### Beispiel 7

| Kapseln enthaltend | |
|---|---|
| DL-α-Tocopherol | 400 mg |
| β-Hydroxyäthylrutosid | 300 mg |
| Vitamin-A-Palmitat | 30.000 I.E. |
| Sojaöl | 100 mg |
| Sojalecithin | 250 mg |

Durch die Verwendung von Lecithin in den Produkten wird der Cholesterinspiegel gesenkt.

## Patentansprüche

1. Verwendung von Vitamin E im Bereich von 200 bis 600 mg pro Darreichungseinheit in Kombination mit Extractum Hippocastani oder β-Hydroxyethylrutosid als alleiniger Wirkstoffkombination sowie gegebenenfalls üblichen Trägern und Hilfsstoffen zur Herstellung eines Mittels zur Behandlung von Erkrankungen der Venen und zur Verbesserung der Durchblutung der Extremitäten, im Wege der oralen Verabreichung.

2. Verwendung nach Anspruch 1, gekennzeichnet durch einen Gehalt an Vitamin E im Bereich von 300 bis 500 mg, bevorzugt von 400 bis 500 mg pro Darreichungseinheit.

3. Verwendung nach einem der Ansprüche 1 oder 2 in Form von Kapseln.

4. Verwendung nach einem der Ansprüche 1 bis 3, dadurch gekennzeichnet, daß das Mittel zusätzlich ein oder mehrere Antikoagulant(ien) oder Vitamin A und/oder einen oder mehrere Emulgatoren und/oder Lecithin enthält.

5. Verwendung nach einem der Ansprüche 1 bis 4, dadurch gekennzeichnet, daß das Mittel zusätzlich Benzaron und/oder dessen Derivate sowie Vitamin A enthält.

6. Verwendung nach einem der Ansprüche 1 bis 5, dadurch gekennzeichnet, daß das Mittel als Emulgatoren Tween® 80 und/oder Ölsäureoleylester in Mengen von 0,1 bis 13 %, vorzugsweise in Mengen von 1 bis 10 %, besonders bevorzugt in Mengen von 1 bis 5 %, enthält.

7. Verwendung nach einem der Ansprüche 1 bis 6, dadurch gekennzeichnet, daß das Mittel zusätzlich Lecithin in Mengen von 0,4 bis 50 %, bevorzugt in Mengen von 1 bis 13 % und gegebenenfalls zusätzlich zu Lecithin 0,4 bis 5 % eines Emulgators enthält.

8. Verwendung nach einem der Ansprüche 1 bis 7, dadurch gekennzeichnet, daß das Mittel Vitamin E natürlichen oder synthetischen Ursprungs in Form von D-α-Tocopherol, D-L-α-Tocopherol und/oder deren Estern, bevorzugt deren Acetaten und/oder Succinaten, enthält.

## Claims

1. The use of vitamin E in the range of from 200 to 600 mg per administration unit in combination with Extractum hippocastani or β-hydroxyethylrutoside as the sole combination of active substances as well as optionally with usual carriers and adjuvants to prepare a drug for the treatment of diseases of the veins and for the improvement of circulation through the extremities via oral administration.

2. The use according to claim 1, characterized by a content of vitamin E in the range of from 300 to 500 mg, preferably of from 400 to 500 mg, per administration unit.

3. The use according to any of claims 1 or 2 in the form of capsules.

4. The use according to any of claims 1 to 3, characterized in that the drug additionally contains one or more anticoagulants or vitamin A and/or one or more emulsifiers and/or lecithin.

5. The use according to any of claims 1 to 4, characterized in that the drug additionally contains benzarone and/or derivatives thereof as well as vitamin A.

6. The use according to any of claims 1 to 5, characterized in that the drug contains Tween® 80 and/or oleyl oleate as emulsifiers in amounts of from 0.1 to 13%, preferably in amounts of from 1 to 10%, more preferably in amounts of from 1 to 5 %.

7. The use according to any of claims 1 to 6, characterized in that the drug additionally contains lecithin in amounts of from 0.4 to 50%, preferably in amounts of from 1 to 13%, and optionally in addition to lecithin from 0.4 to 5% of an emulsifier.

8. The use according to any of claims 1 to 7, characterized in that the drug contains vitamin E from natural or artificial sources in the form of D-α-tocopherol, D-L-α-tocopherol and/or esters thereof, preferably acetates and/or succinates thereof.

## Revendications

1. Utilisation de la vitamine E à raison de 200 à 600 mg par unité administrée, en combinaison avec de l'extrait de marron d'Inde (Extractum Hippocastani) ou du β-hy-droxyéthylrutoside, comme combinaison unique de principes actifs, ainsi qu'éventuellement des supports et des adjuvants usuels, pour la préparation d'un agent pour le traitement des maladies des veines et pour l'amélioration de la circulation sanguine dans les extrémités, par administration orale.

2. Utilisation selon la revendication 1, caractérisée par une teneur en vitamine E comprise entre 300 et 500 mg, du préférence entre 400 et 500 mg par unité administrée.

3. Utilisation selon la revendication 1 ou 2 sous la forme de capsules.

4. Utilisation selon l'une des revendications 1 à 3, caractérisée en ce que l'agent contient, en outre, un ou plusieurs anticoagulants ou de la vitamine A et/ou un ou plusieurs émulslfiants et/ou de la lécithine.

5. Utilisation selon l'une des revendications 1 à 4, caractérisée an ce que l'agent contient, en outre, de la bensarone et/ou ses dérivés, ainsi que de la vitamine A.

6. Utilisation selon l'une des revendications 1 à 5, caractérisée en ce que l'agent contient, comme émulsifiants, du Tween® 80 et/ou de l'oléate d'oléyle en des quantités de 0,1 à 13 %, de préférence en des quantités de 1 à 10 %, et, d'une manière particulièrement préférée, en des quantités de 1 à 5 %.

7. Utilisation selon l'une des revendications 1 à 6, caractérisée en ce que l'agent contient, en outre, de la lécithine en des quantités de 0,4 à 50 %, de préférence en des quantités de 1 à 13 %, et éventuellement 0,4 à 5 % d'un émulsifiant, en plus de la lécithine.

8. Utilisation selon l'une des revendications 1 à 7, caractérisée en ce que l'agent contient de la vitamine E d'origine naturelle ou synthétique sous la forme D-α-tocophérol, D,L-α-tocophérol et/ou leurs esters, de préférence leurs acétates et/ou leurs succinates.
